# EUROPÄISCHE PATENTANMELDUNG

(11) **EP 1 160 237 A2**
(43) Veröffentlichungstag der Anmeldung: **05.12.2001**
(21) Anmeldenummer: 01111927.8
(22) Anmeldetag: 18.05.2001
(51) Int. Cl.: C07C 69/675, C07C 67/31, C07C 59/01, C07C 51/367

(54) **Verfahren zur Herstellung optisch aktiver Trimethylmilchsäure und ihrer Ester**

(30) Priorität: 31.05.2000 DE 10027154
(71) Anmelder: BAYER AG, 51368 Leverkusen (DE)
(72) Erfinder: Sirges, Wolfram, Dr., 40597 Düsseldorf (DE); Dreisbach, Claus, Dr., 51065 Köln (DE)

(57) **Zusammenfassung**

Die Erfindung betrifft ein neues Verfahren zur Herstellung optisch aktiver Trimethylmilchsäure und/oder ihrer Ester durch katalytische Hydrierung von Trimethylbrenztraubensäure und/oder ihrer Ester in Gegenwart von Edelmetallkomplexkatalysatoren mit Phosphor-haltigen Liganden.

## Beschreibung

Die Erfindung betrifft ein neues Verfahren zur Herstellung optisch aktiver Trimethylmilchsäure und/oder ihrer Ester durch katalytische Hydrierung von Trimethylbrenztraubensäure und/oder ihrer Ester.

Optisch aktive Trimethylmilchsäure oder ihre Ester werden z. B. als Bausteine für HIV-Protease-Inhibitoren benötigt (Bioorg. Med. Chem. Lett., 1995, 5, 1729-1734). Ihrer Synthese kommt daher besondere Bedeutung zu.

Es sind bereits verschiedene Synthesewege bekannt: In Biotechnol. Biotech. 1986, 8-13 wird beispielsweise beschrieben, Trimethylbrenztraubensäure enzymatisch mit einer Alkoholdehydrogenase enantioselektiv zu reduzieren. Das Verfahren hat jedoch den Nachteil, dass in Gegenwart eines Cofaktors gearbeitet werden muss, dessen Regenerierung aufwendig und teuer ist.

Weiter sind sowohl chemische (Bull. Chem,. Soc. Jpn., 1968, 41, 2178-2179) als auch biologische Methoden zur Racematspaltung von Trimethylmilchsäure beschrieben (Appl. Environ. Microbio., 1983, 45, 884-891). Der Nachteil dieser Methoden ist, dass die maximale Ausbeute für das Zielenantiomere, wie bei Racematspaltungen üblich, 50 % beträgt und das Fehlenantiomere meist verworfen werden muß.

Eine weitere Methode ist die Diazotierung von tert.-Leucin unter anschließender Hydrolyse der Diazoniumverbindung mit Wasser (Chem. Ber., 1991,124, 849-859). Dieses Verfahren benötigt jedoch das sehr teure enantiomerenreine tert.-Leucin und führt durch unerwünschte Umlagerungsreaktionen zu Nebenprodukten und ist daher unwirtschaftlich.

Aus J. Org. Chem., 1988, 53, 1231-1238 und J. Org. Chem., 1986, 51, 3396-3398 ist die Herstellung von enantiomerenreinen Trimethylmilchsäureestern durch Reduktion von Trimethylbrenztraubensäureestern mit chiral modifizierten Boran-Reagenzien bekannt. Dieses Verfahren hat aber den Nachteil, dass stöchiometrische Mengen des teuren und kompliziert zu synthetisierenden Boran-Reagenzes benötigt werden.

Aus EP-A-0 901 997 ist ein Verfahren zur Herstellung optisch aktiver Alkohole durch asymmetrische Hydrierung von Ketonen bekannt. Das Verfahren ist jedoch ausschließlich auf aliphatische oder aliphatisch/aromatische Ketone beschränkt, wobei in Gegenwart von Übergangsmetallkomplexkatalysatoren, einer Base sowie einem Diamin hydriert wird. Die Übergangsmetallkomplexkatalysatoren enthalten Bisphosphinliganden.

Aus EP-A-0 643 065 sind weitere spezielle Bisphosphine bekannt, die in Form ihrer Komplexe mit Metallen der Gruppe VIII, insbesondere Ruthenium für asymmetrische Hydrierungen eingesetzt werden können Als geeignete Substrate werden allgemein substituierte oder unsubstituierte α- oder β-Ketoester, α- oder β-Keto-Amide, α- oder β-Amino- oder α- oder β-Hydroxy-Ketone und Acetamidozimtsäurederivate genannt. Der Fokus der Anwendung liegt auf der asymmetrischen Hydrierung von 2-Arylpropensäuren.

Ferner werden in EP-A-0 564 406 Ferrocenyldiphosphine als Liganden für homogene Rhodium- und Iridium-Katalysatoren beschrieben, die zur asymmetrischen Hydrierung von prochiralen Verbindungen mit Kohlenstoff-Kohlenstoff und Kohlenstoff-Heteroatom-Doppelbindungen eingesetzt werden. Beispiele für solche Verbindungen sind prochirale Olefine, Enamine, Imine und Ketone.

Für den sterisch anspruchsvollen Phenylbrenztraubensäuremethylester wird in Tetrahedron:Asymmetry 5, 675-690 eine asymmetrische Hydrierung in Gegenwart von verschiedenen Phosphin-Liganden beschrieben, die zwar überwiegend zu sehr hohen Ausbeuten führen, gleichzeitig aber nur unbefriedigende Enantiomerenüberschüssen liefern, die teilweise im Bereich von lediglich 27 bzw. 30 % ee liegen.

Die Aufgabe der vorliegenden Erfindung bestand somit darin, ein neues Verfahren bereitzustellen, welches die enantioselektive Herstellung optisch aktiver Trimethylmilchsäure und ihrer Ester mit hohen Ausbeuten ermöglicht und keine Verwendung teurer Reagenzien erfordert.

Gegenstand der Erfindung ist ein Verfahren zur Herstellung von optisch aktiver Trimethylmilchsäure und/oder ihrer Ester der allgemeinen Formel (I) 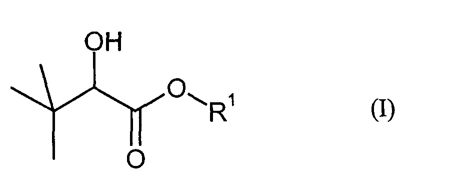 wobei R¹ für Wasserstoff, Alkyl, Aryl, Aralkyl oder Heteroaryl steht,
durch enantioselektive Hydrierung von Trimethylbrenztraubensäure und/oder deren Ester der allgemeinen Formel (II) 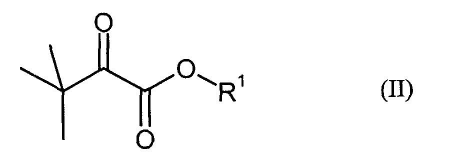 wobei R¹ die für die allgemeine Formel (I) genannten Bedeutungen besitzt,
in Gegenwart eines Katalysators, dadurch gekennzeichnet, dass es sich bei dem Katalysator um Edelmetallkomplexe handelt, die optisch aktive Bisphosphine als Liganden enthalten.

Das erfindungsgemäße Verfahren ermöglicht die enantiomerenreine Herstellung von Trimethylmilchsäure und/oder ihrer Ester der allgemeinen Formel (I), wobei der Rest R¹ für H, Alkyl, Aryl, Aralkyl oder Heteroaryl steht. Die Alkylreste in den vorgenannten Substituenten können dabei jeweils geradkettig oder verzweigt sein.

Bevorzugt steht der Rest R¹ für H, C₁-C₂₀-Alkyl, C₆-C₁₄-Aryl, C₇-C₁₅-Aralkyl oder C₂-C₁₂-Heteroaryl. Besonders bevorzugt steht R¹ für H, C₁-C₁₀-Alkyl, C₆-C₁₀-Aryl, C₇-C₁₁-Aralkyl oder C₂-C₉-Heteroalkyl und insbesondere für Wasserstoff, Methyl, Ethyl, Propyl, Isopropyl, Butyl, Isobutyl, Sec.-Butyl, Pentyl, Neopentyl, Isopentyl, Phenyl, Benzyl, Naphthyl, 2-Furyl, 3-Furyl, 2-Pyrrolyl und 3-Pyrrolyl.

Die Alkyl-, Aryl-, Aralkyl- und Heteroalkylreste können darüberhinaus auch noch weiter substiutiert sein durch Cl, Br, F, I, C₁-C₄-Alkoxy oder C₁-C₄-Alkyl.

Im erfindungsgemäßen Verfahren können beispielsweise Katalysatoren mit den folgenden enantiomerenreinen Bisphosphinen der allgemeinen Formeln (B1)- (B15) eingesetzt werden:

Ein Bisphosphin der allgemeinen Formel (B1) 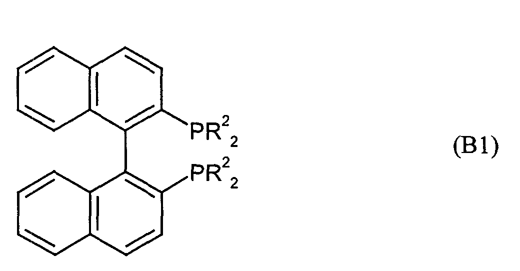 wobei
- R²: Phenyl, 3-Methylphenyl, 4-Methylphenyl, 3,5-Dimethylphenyl, 4-Methoxyphenyl, 3,5-Dimethyl-4-methoxyphenyl, Cyclohexyl oder Cyclopentyl bedeutet,
oder der allgemeinen Formel (B2) 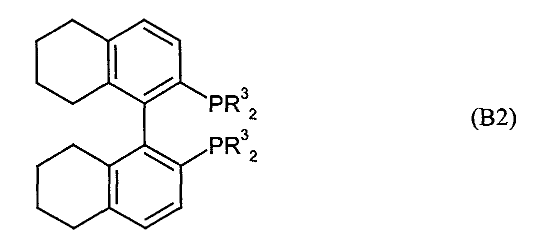 wobei
- R³: Phenyl, 4-Methylphenyl, 3,5-Dimethylphenyl, 4-Methoxyphenyl, 3,5-Dimethyl-4-methoxyphenyl oder Cyclohexyl bedeutet,
oder der allgemeinen Formel (B3) 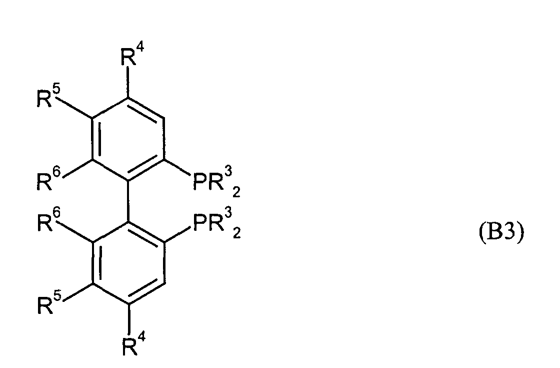 wobei
- R³: Phenyl, 4-Methylphenyl, 3,5-Dimethylphenyl, 4-Methoxyphenyl, 3,5-Dimethyl-4-methoxyphenyl oder Cyclohexyl,
- R⁴: H, Methyl oder Methoxy,
- R⁵: H, Methyl, Methoxy oder Chlor und
- R⁶: Methyl, Methoxy oder Trifluoromethyl bedeutet,
oder der allgemeinen Formel (B4) 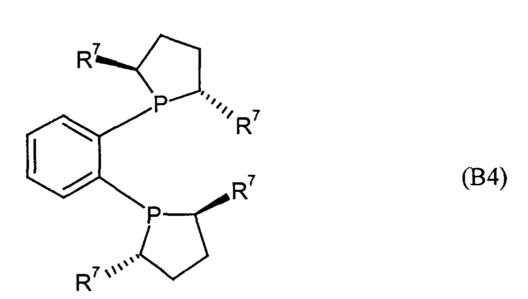 wobei
- R⁷: für Methyl, Ethyl, Propyl oder Isopropyl steht,
oder 2,3-Bis-(diphenylphosphino)butan der Formel (B5) 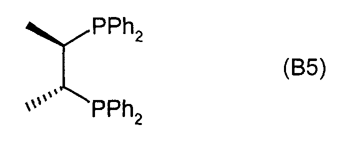 oder 1,2-Bis-(diphenylphosphino)propan der Formel (B6) 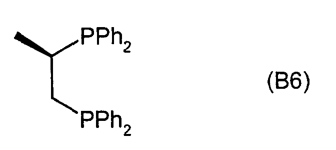 oder 5,6-Bis-(diphenylphosphino)-2-norbornan der Formel (B7) 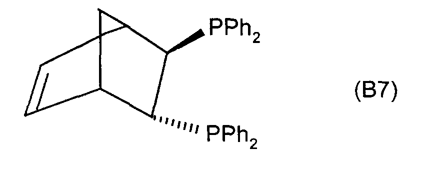 oder 1-substituiertes 3,4-Bis-(diphenylphosphino)pyrrolidin der Formel (B8) 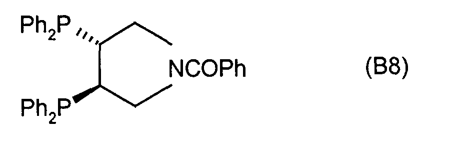 oder 2,4-Bis-(diphenylphosphino)pentan der Formel (B9) 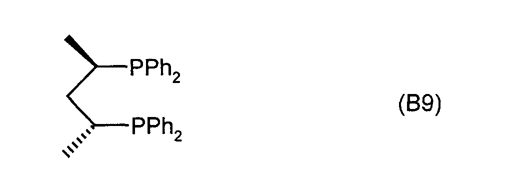 oder 2,3-O-isopropyliden-2,3-dihydroxy-1,4-bis-(diphenylphosphino)butan der Formel (B 10) 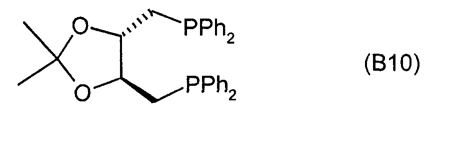 oder 1,2-Bis-[(o-methoxyphenyl)phenylphosphino]ethan der allgemeinen Formel (B11) 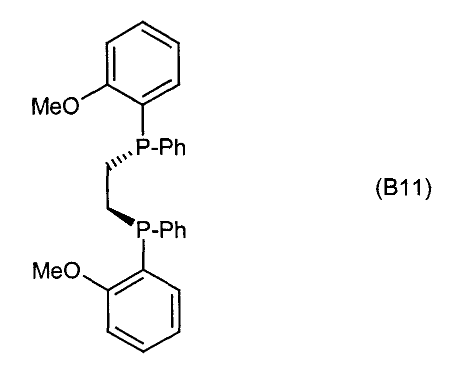 oder 1-[1',2-Bis-(diphenylphosphino)ferrocenyl]ethanol der Formel (B12) 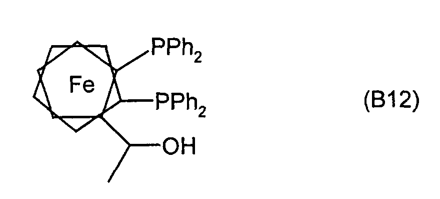 oder 1-tert.Butoxycarbonyl-4-diphenylphosphino-2-diphenylphosphinomethylpyrrolidin der Formel (B13) 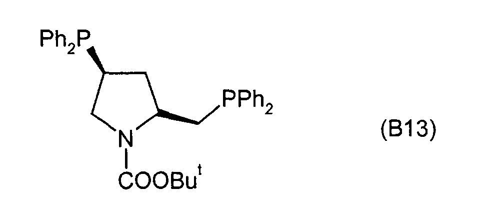 oder der allgemeinen Formel (B14) 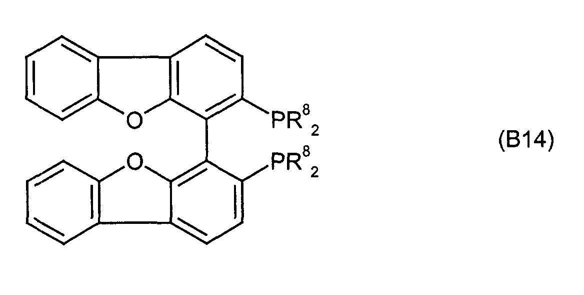 wobei
- R⁸: Phenyl, Cyclohexyl, 4-Methylphenyl, 4-Methoxyphenyl, 3,5-Dimethylphenyl, 3,5-Dimethyl-4-methoxyphenyl, 4-tert.-Butyl oder 3,5-di-tert.-Butyl bedeutet,
oder Ferrocenyldiphosphine der allgemeinen Formel (B15) 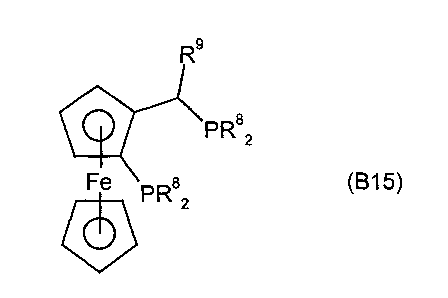 wobei
- R⁸: die für Formel B 14 angegebene Bedeutung hat und
- R⁹: C₁-C₈-Alkyl, Phenyl oder 1- 3fach mit C₁ - C₄-Alkoxy substituiertes Phenyl bedeutet.

Geeignete Bisphosphine der vorgenannten Formel (B1) sind:
2,2µ-Bis-(diphenylphosphino)-1,1µ-binaphthyl,
2,2µ-Bis-(di-4-tolylphosphino)-1,1µ-binaphthyl
- beschrieben in J. Org. Chem. 1986, 51, 629.

Geeignete Bisphosphine der vorgenannten Formel (B3) sind:
(5,5'-Dichloro-6,6'-dimethoxy-biphenyl-2,2'-diyl)-bis-diphenylphosphin
- beschrieben in EP-A 0 749 973
(4,4',6,6'-Tetramethyl-5,5 '-dimethoxybiphenyl-2,2'-diyl)-bis(diphenylphosphin)
(4,4',6,6'-Tetramethyl-5,5 '-dimethoxybiphenyl-2,2'-diyl)-bis(di-p-methoxyphenylphosphin)
- beschrieben in Chem. Pharm. Bull. 1991, 39, 1085
(4,4',6,6'-Tetratrifluoromethylbiphenyl-2,2'-diyl)-bis(diphenylphosphin)
(4,6-Ditrifluoromethyl-4',6'-dimethyl-5'-methoxybiphenyl-2,2'-diyl)-bis(diphenylphosphin)
- beschrieben in Synlett 1991, 827
(2-Dicyclohexyl-2'-diphenylphosphino-4,4',6,6'-tetramethyl-5,5'-dimethoxybiphenyl-2,2'-diyl)-bis(diphenylphosphin)
- beschrieben in Tetrahedron: Asymmetry 1992, 3, 13
(6,6'-Dimethyl-2,2'-biphenylen)-bis(diphenylphosphin)
(4,4',6,6'-Tetramethyl-2,2'-biphenylen)-bis(diphenylphosphin)
(3,3',6,6'-Tetramethyl-2,2'-biphenylen)-bis(diphenylphosphin)
(4,4'-Difluoro-6,6'-dimethyl-2,2'-biphenylen)-bis(diphenylphosphin)
(4,4'-bis(Dimethylamino)-6,6'-dimethyl-2,2'-biphenylen)-bis(diphenylphosphin)
(6,6'-Dimethyl-2,2'-biphenylen)-bis(di-p-tolylphosphin)
(6,6'-Dimethyl-2,2'-biphenylen)-bis (di-o-tolylphosphin)
(6,6'-Dimethyl-2,2'-biphenylen)-bis(di-m-fluorophenylphosphin)
1,1 1-bis(Diphenylphosphino)-5,7-dihydrodibenzo[c,e]oxepin
- beschrieben in JP-B-4-15796 (wobei "B" bedeutet: geprüfte Japanische Patentanmeldung)
(6,6'-Dimethoxybiphenyl-2,2'-diyl)-bis(diphenylphosphin)
(5,5',6,6'-Tetramethoxybiphenyl-2,2'-diyl)-bis(diphenylphosphin)
(6,6'-Dimethoxybiphenyl-2,2'-diyl)-bis(di-p-tolylphosphin) und
(4,4',5,5',6,6'-Hexamethoxybiphenyl-2,2'-diyl)-bis(diphenylphosphin)
- beschrieben in JP-A-3-5492.

Geeignete Bisphosphine der Formel (B4) sind:
1,2-Bis(2,5-dimethylphosphorano)benzol
1,2-Bis(2,5-diethylphosphorano)benzol
1,2-Bis(2,5-dipropylphosphorano)benzol und
1,2-Bis(2,5-diisopropylphosphorano)benzol
- beschrieben in J.Am. Chem. Soc. 1993, 115, 10125.

Als Edelmetallkomplexkatalysatoren sind im erfindungsgemäßen Verfahren besonders solche auf der Basis von Ruthenium, Rhodium und Iridium bevorzugt. Besonders bevorzugt werden Rutheniumkomplexkatalysatoren eingesetzt.

Geeignet sind beispielsweise die folgenden durch die allgemeinen Formeln (III)-(X) definierten Rutheniumkomplexe optisch aktiver Bisphosphine, ohne darauf beschränkt zu sein:

Ru₂Cl₄B₂(S) (III)

[Ru Hal Q B]⁺ Y⁻ (IV)

Ru Bₙ OOCR¹⁰OOCR¹¹ (V)

[Ru Hₓ Bₙ]^{m+} Y ^{m-} (VI)

[Ru Hal (PR¹² ₂R¹³)B]⁽²⁺⁾ Hal⁻ ₂ (VII)

[Ru H Hal B₂] (VIII)

[B Ru (acac)₂] (IX)

[B Ru Y₂] (X)

worin
- acac: Acetylacetonat bedeutet und
- B: für ein Bisphosphin der allgemeinen Formeln (B1)-(B15) steht,
- Hal: für Halogen, insbesondere Chlor, Brom oder Iod steht,
- R¹⁰ und R¹¹: gleich oder verschieden sind und für C₁-C₉-Alkyl, vorzugsweise C₁-C₄-Alkyl, welches gegebenenfalls substituiert ist durch Halogen, insbesondere Fluor, Chlor oder Brom, stehen oder Phenyl, welches gegebenenfalls durch C₁-C₄-Alkyl substituiert ist, bedeuten oder für eine α-Aminoalkylsäure mit vorzugsweise bis zu 4 C-Atomen stehen, oder R¹⁰ und R¹¹ gemeinsam eine Alkylidengruppe mit bis zu 4 C-Atomen bilden,
- R¹² und R¹³: gleich oder verschieden sind und für gegebenenfalls substituiertes Phenyl stehen, vorzugsweise substituiert durch C₁-C₄-Alkyl oder Halogen,
- Y: für Halogen, ClO₄, BF₄ oder PF₆ steht
- Q: für einen unsubstituierten oder substituierten Benzolring, bevorzugt p-Cymol, steht,
- S: für ein tertiäres Amin, bevorzugt Triethylamin, Tri-n-Butylamin oder Pyridin steht,
- n und m: gleich oder verschieden sind und gleich 1 oder 2 sind
- x: für 0 oder 1 stehen
wobei in Formel (VI) n für 1 und m für 2 steht, wenn x = 0 ist, und n für 2 und m für 1 steht, wenn x = 1 ist.

Die Komplexe der allgemeinen Formeln (III) bis (IX) können nach an sich bekannten Methoden hergestellt werden.

Die Komplexe der Formeln (III) und (VIII) lassen sich z. B. in analoger Weise gemäß den Verfahren herstellen, die in EP-A-0 174 057 oder in J. Chem Soc. Chem. Comm. 922 (1985) beschrieben werden.

Die Komplexe der allgemeinen Formel (IV) ergeben sich z. B. durch Umsetzung von bekannten Rutheniumkomplexen [RuHal₂Q]₂ mit Bisphosphinen der allgemeinen Formel (B1) in inerten organischen Lösungsmitteln, wie z. B. in EP-A-0 366 390 oder EP-A-0 749 973 beschrieben.

Komplexe der allgemeinen Formel (V) mit n = 1 können z. B. erhalten werden nach Verfahren, die in EP-A-0 245 959 angegeben sind, indem man Komplexe der allgemeinen Formel (III) mit den entsprechenden Carbonsäuren umsetzt.

Komplexe der allgemeinen Formel (V) mit n = 2 oder mit n = 1 und R¹⁰, R¹¹ = CF₃ können nach den in EP-A-0 272 787 angegebenen Verfahren hergestellt werden.
Die Komplexe der allgemeinen Formel (VI) können hergestellt werden analog dem Verfahren gemäß EP-A-0 256 634.

Die Komplexe der allgemeinen Formel (VII) können hergestellt werden analog dem Verfahren gemäß EP-A-0 470 756.

Komplexe der allgemeinen Formel (IX) können analog den in Organometallics 1993, 1467 angegebenen Verfahren hergestellt werden.

Die Komplexe der Formel (X) können analog zu den in J. Am. Chem. Soc., 1987, 109, 5856-5858 oder in Tetrahedron: Asymmetry, 5, 1994, 675-690 angegebenen Verfahren hergestellt werden.

Auch die Herstellung von Komplexen auf der Basis von Rhodium oder Iridium kann nach an sich bekannten Methoden erfolgen, indem man beispielsweise das entsprechende Bisphosphin mit einer Verbindung, die Rhodium oder Iridium abgeben kann, in einem geeigneten, inerten organischen oder wässrigen Lösungsmittel umsetzt.

Als Rhodium abgebende Verbindungen können beispielsweise eingesetzt werden: organische Rhodium-Komplexe mit Ethylen oder Propylen sowie mit Bis-Olefinen wie 1,5-Cyclooctadien, 1,5-Hexadien, Bicyclo(2.2.1)hepta-2,5-dien oder mit weiteren Dienen, die mit Rhodium leicht lösliche Komplexe bilden. Bevorzugte, Rhodium abgebende Verbindungen sind z.B. Di-chloro-bis-(1,5-cyclooctadien)dirhodium, Dichloro-bis(norbornadien)dirhodium, Bis-(1,5-cyclococtadien)-rhodium-tetrafluorborat oder Bis(cyclooctadien)-rhodium-perchlorat. Als Iridium abgebende Verbindung kann beispielsweise Di-chloro-bis-(1,5-cyclooctadien)diiridium genannt werden.

Bei der Durchführung des erfindungsgemäßen Verfahrens können diese KatalysatorKomplexe zuerst hergestellt und gegebenenfalls isoliert und dann zu einer Lösung der Edukte der allgemeinen Formel (II) gegeben werden. Alternativ können sie jedoch auch in situ hergestellt werden, d.h. bereits in Gegenwart der Edukte der allgemeinen Formel (II).

Bei der Herstellung der Katalysatorkomplexe liegt das Verhältnis von Metallen zu Bisphosphinen der allgemeinen Formel B1-B15 zweckmäßigerweise im Bereich von 0,5 bis 2 Mol, vorzugsweise im Bereich von 0,9 bis 1,1 Mol Ruthenium pro Mol Bisphosphin-Ligand. Das Verhältnis von Metall in den Komplexen zu den Verbindungen der Formel (II) liegt üblicherweise im Bereich von 1 : 10 bis 1 : 10⁶ und vorzugsweise im Bereich von 1: 30 bis 1 : 10⁵.

Die enantioselektive Hydrierung kann in einem geeigneten, unter den Reaktionsbedingungen inerten organischen Lösungsmittel erfolgen. Als derartige Lösungsmittel sind z. B. niedere Alkohole mit 1 bis 6 C-Atomen, oder Gemischen derartiger Alkohole mit halogenierten Kohlenwasserstoffen wie Methylenchlorid oder Chloroform, oder mit gegebenenfalls cyclischen Ethern wie Diethylether, Tetrahydrofuran oder Dioxan, oder mit Ketonen wie Aceton, Methylethylketon oder Methylisobutylketon geeignet. Ebenfalls geeignet als Mischungspartner sind aliphatische Kohlenwasserstoffe, wie Hexan und Heptan, cycloaliphatische Kohlenwasserstoffe, wie Cyclohexan und Methylcyclohexan, oder aromatische Kohlenwasserstoffe, wie Toluol und Benzol. Die Mischungspartner können gegebenenfalls auch in reiner Form eingesetzt werden.

Die enantioselektive Hydrierung von Verbindungen der allgemeinen Formel (II) mit optisch aktiven Bisphosphin-Katalysatoren erfolgt zweckmäßigerweise bei einer Temperatur im Bereich von 0-150°C, vorzugsweise im Bereich von 15-100°C.

Der Druck liegt im Bereich von 1 - 250 bar Wasserstoff, vorzugsweise im Bereich von 5 - 200 bar, und besonders bevorzugt ist ein Wasserstoffdruck im Bereich von 20 - 180 bar.

Das erfindungsgemäße Verfahren zeichnet sich durch eine sehr gute Ausbeute und eine gleichzeitig hohe Enantioselektivität aus. Die Verwendung eines Cofaktors ― wie im Fall der enzymatischen Reduktion mit einer Alkoholdehydrogenase ― ist nicht erforderlich. Auch der Einsatz teurer und kompliziert zu synthetisierender Boran-Reagenzien ist nicht notwendig. Es ist überraschend, dass die für andere asymmetrische Hydrierungen bereits bekannten optisch aktiven Bisphosphin-Liganden auch im Fall der hier eingesetzten sterisch sehr anspruchsvollen Trimethylbrenztraubensäure(ester) zu einer exzellenten Enantioselektivität führen. Erreicht werden Enantiomerenüberschüsse, die bei 40% ee oder mehr liegen. Bevorzugt werden Enantiomerenüberschüssen von ≥ 90 %ee erreicht und insbesondere von ≥ 95 %ee.

### Beispiele:

Die Enantiomeren von (5,5'-Dichloro-6,6'-dimethoxy-biphenyl-2,2'-diyl)-bis-(diphenylphosphin) werden gemäß den Beispielen 3-7 der EP-A-0 749 973 hergestellt.

Trimethylbrenztraubensäure ist kommerziell erhältlich. Ihre Ester können nach an sich bekannten Verfahren durch saure Veresterung aus der Trimethylbrenztraubensäure erhalten werden. Enantiomerenüberschüsse werden, wenn nicht anders angegeben, mittels Gaschromatographie (GC) ermittelt.

### Beispiel 1

15 ml destilliertes Aceton wird in einem Kolben unter Argon (5.0) vorgelegt, unter Rühren evakuiert und mit Argon belüftet. Dieser Vorgang wird 3 mal wiederholt. Dann werden unter Rühren und im Argon-Gegenstrom 61,5 mg (R)-(+)-2,2µ-Bis(diphenylphosphino)-1,1µ-binaphthyl (Lieferant: Acros) und 30,8 mg Bis-(2-Methylallyl)cycloocta-1,5-dien Ruthenium (III) Komplex (Lieferant: Acros) in dem Aceton gelöst. Zu der entstandenen Suspension werden 0,84 ml Bromwasserstoff (w (HBr) = 48%) zugegeben und zweimal unter Rühren evakuiert und mit Argon belüftet. Dann wird 0,5 h unter Argon nachgerührt. Es entsteht eine orange-rote Lösung.

In einem weiteren Kolben werden 0,52 g Trimethylbrenztraubensäuremethylester und 50 ml destilliertes Methanol unter Argon vorgelegt. Diese Lösung wird dreimal unter Rühren evakuiert und mit Argon belüftet. Dann wird die Katalysatorlösung durch eine Hohlnadel aus dem ersten Kolben mit Argon in den zweiten Kolben umgedrückt. Die dabei entstandene gelbe trübe Lösung wird dann in einen 0,3L V₄A-Autoklav, der zuvor fünfmal mit 100 bar Stickstoff (5.0) gespült worden ist, mit Argon umgedrückt.

Unter Rühren (1000U/min) wird der Autoklav auf 50°C erwärmt. Bei 50°C werden 150 bar Wasserstoff aufgedrückt und 24 Stunden unter diesen Bedingungen hydriert.

Anschließend wird der Autoklav auf Raumtemperatur abgekühlt, entspannt und mit Stickstoff gespült.

Gemäß GC ist die Ausbeute an Trimethylmilchsäuremethylester quantitativ und der Enantiomerenüberschuß des R-Enantiomeren beträgt 97,8 %.

### Beispiel 2

15 ml destilliertes Aceton werden in einem Kolben unter Argon (5.0) vorgelegt, unter Rühren evakuiert und mit Argon belüftet. Dieser Vorgang wird dreimal wiederholt. Dann werden unter Rühren und im Argon-Gegenstrom 49,9 mg (4S,5S)-(+)-4,5-Bis-(Diphenylphosphinomethyl)-2,3-Dimethyl-1,3-Dioxolan (Lieferant: Aldrich) und 30,8 mg Bis-(2-Methylallyl)cycloocta-1,5-dien Ruthenium (III) Complex (Lieferant: Acros) in dem Aceton gelöst. Zu der entstandenen Suspension werden 0,84 ml Bromwasserstoff (w (HBr) = 48%) zugegeben und zweimal unter Rühren evakuiert und mit Argon belüftet. Dann wird 0,5 h unter Argon nachgerührt. Es entsteht eine orange-rote Lösung.

In einem weiteren Kolben werden 0,52 g Trimethylbrenztraubensäuremethylester und 50 ml destilliertes Methanol unter Argon vorgelegt. Diese Lösung wird dreimal unter Rühren evakuiert und mit Argon belüftet. Dann wird die Katalysatorlösung durch eine Hohlnadel aus dem ersten Kolben mit Argon in den zweiten Kolben umgedrückt. Die dabei entstandene gelbe trübe Lösung wird dann in einen 0,3L V₄A-Autoklav, der zuvor fünfmal mit 100 bar Stickstoff (5.0) gespült worden ist, mit Argon umgedrückt.

Unter Rühren (1000U/min) wird der Autoklav auf 50°C erwärmt. Bei 50°C werden 150 bar Wasserstoff aufgedrückt und 24 Stunden unter diesen Bedingungen hydriert. Anschließend wird der Autoklav auf Raumtemperatur abgekühlt, entspannt und mit Stickstoff gespült.

Gemäß GC beträgt die Ausbeute an Trimethylmilchsäuremethylester 37 % und der Enantiomerenüberschuß des R-Enantiomeren 44 %.

### Beispiel 3

15 ml destilliertes Aceton werden in einem Kolben unter Argon (5.0) vorgelegt, unter Rühren evakuiert und mit Argon belüftet. Dieser Vorgang wird dreimal wiederholt. Dann werden unter Rühren und im Argon-Gegenstrom 42,6 mg (2S,3S)-(-)-Bis-(Diphenylphosphino)-butan (Lieferant: Aldrich) und 30,8 mg Bis-(2-Methylallyl)cycloocta-1,5-dien Ruthenium (III) Komplex (Lieferant: Acros) in dem Aceton gelöst. Zu der entstandenen Suspension werden 0,84 ml Bromwasserstoff (w (HBr) = 48%) zugegeben und zweimal unter Rühren evakuiert und mit Argon belüftet. Dann wird 0,5 h unter Argon nachgerührt. Es entsteht eine orange-rote Lösung.

In einem weiteren Kolben werden 0,52 g Trimethylbrenztraubensäuremethylester und 50 ml destilliertes Methanol unter Argon vorgelegt. Diese Lösung wird dreimal unter Rühren evakuiert und mit Argon belüftet. Dann wird die Katalysatorlösung durch eine Hohlnadel aus dem ersten Kolben mit Argon in den zweiten Kolben umgedrückt. Die dabei entstandene gelbe trübe Lösung wird dann in einen 0,3L V₄A-Autoklav, der zuvor fünfmal mit 100bar Stickstoff (5.0) gespült worden ist, mit Argon umgedrückt.

Unter Rühren (1000U/min) wird der Autoklav auf 50°C erwärmt. Bei 50°C werden 150 bar Wasserstoff aufgedrückt und 24 Stunden unter diesen Bedingungen hydriert. Anschließend wird der Autoklav auf Raumtemperatur abgekühlt, entspannt und mit Stickstoff gespült.

Gemäß GC beträgt die Ausbeute an Trimethylmilchsäuremethylester 16 % und der Enantiomerenüberschuß des R-Enantiomeren 53,4 %.

### Beispiel 4

15 ml destilliertes Aceton werden in einem Kolben unter Argon (5.0) vorgelegt, unter Rühren evakuiert und mit Argon belüftet. Dieser Vorgang wird dreimal wiederholt. Dann werden unter Rühren und im Argon-Gegenstrom 65,1 mg (+)-(5,5'-Dichloro-6,6'-dimethoxy-biphenyl-2,2'-diyl)-bis-(diphenylphosphin) und 30,8 mg Bis-(2-Methylallyl)cycloocta-1,5-dien Ruthenium (III) Komplex (Lieferant: Acros) in dem Aceton gelöst. Zu der entstandenen Suspension werden 0,84 ml Bromwasserstoff (w (HBr) = 48%) zugegeben und zweimal unter Rühren evakuiert und mit Argon belüftet. Dann wird 0,5 h unter Argon nachgerührt. Es entsteht eine orange-rote Lösung.

In einem weiteren Kolben werden 0,52 g Trimethylbrenztraubensäuremethylester und 50 ml destilliertes Methanol unter Argon vorgelegt. Diese Lösung wird dreimal unter Rühren evakuiert und mit Argon belüftet. Dann wird die Katalysatorlösung durch eine Hohlnadel aus dem ersten Kolben mit Argon in den zweiten Kolben umgedrückt. Die dabei entstandene gelbe trübe Lösung wird dann in einen 0,3L V₄A-Autoklav, der zuvor fünfmal mit 100 bar Stickstoff (5.0) gespült worden ist, mit Argon umgedrückt.

Unter Rühren (1000U/min) wird der Autoklav auf 50°C erwärmt. Bei 50°C werden 150 bar Wasserstoff aufgedrückt und 24 Stunden unter diesen Bedingungen hydriert. Anschließend wird der Autoklav auf Raumtemperatur abgekühlt, entspannt und mit Stickstoff gespült.

Gemäß GC beträgt die Ausbeute an Trimethylmilchsäuremethylester 97,5 % und der Enantiomerenüberschuß des R-Enantiomeren ist > 98 %.

### Beispiel 5

15 ml destilliertes Aceton werden in einem Kolben unter Argon (5.0) vorgelegt, unter Rühren evakuiert und mit Argon belüftet. Dieser Vorgang wird dreimal wiederholt.

Dann werden unter Rühren und im Argon-Gegenstrom 65,1 mg (+)-(5,5'-Dichloro-6,6'-dimethoxy-biphenyl-2,2'-diyl)-bis-(diphenylphosphin) und 30,8 mg Bis-(2-Methylallyl)cycloocta-1,5-dien Ruthenium (III) Komplex (Lieferant: Acros) in dem Aceton gelöst. Zu der entstandenen Suspension werden 0,84 ml Bromwasserstoff (w (HBr) = 48%) zugegeben und zweimal unter Rühren evakuiert und mit Argon belüftet. Dann wird 0,5 h unter Argon nachgerührt. Es entsteht eine orange-rote Lösung.

In einem weiteren Kolben werden 5,2 g Trimethylbrenztraubensäuremethylester und 50 ml destilliertes Methanol unter Argon vorgelegt. Diese Lösung wird dreimal unter Rühren evakuiert und mit Argon belüftet. Dann wird die Katalysatorlösung durch eine Hohlnadel aus dem ersten Kolben mit Argon in den zweiten Kolben umgedrückt. Die dabei entstandene gelbe trübe Lösung wird dann in einen 0,3L V₄A-Autoklav, der zuvor fünfmal mit 100 bar Stickstoff (5.0) gespült worden ist, mit Argon umgedrückt.

Unter Rühren (1000U/min) wird der Autoklav auf 50°C erwärmt. Bei 50°C werden 150 bar Wasserstoff aufgedrückt und 24 Stunden unter diesen Bedingungen hydriert. Anschließend wird der Autoklav auf Raumtemperatur abgekühlt, entspannt und mit Stickstoff gespült. Danach wird die Hydrierlösung von den Lösungsmitteln befreit und destilliert.

Die isolierte Ausbeute beträgt 3 g (58 % der Theorie). Gemäß GC ist der Gehalt an (R)-Trimethylmilchsäuremethylester > 99 % mit einem Enantiomerenüberschuss größer > 98 % ee.

### Beispiel 6

15 ml destilliertes Aceton werden in einem Kolben unter Argon (5.0) vorgelegt, unter Rühren evakuiert und mit Argon belüftet. Dieser Vorgang wird dreimal wiederholt. Dann werden unter Rühren und im Argon-Gegenstrom 13 mg (+)-(5,5'-Dichloro-6,6'-dimethoxy-biphenyl-2,2'-diyl)-bis-(diphenylphosphin) und 6,2 mg Bis-(2-Methylallyl)cycloocta-1,5-dien Ruthenium (III) Komplex (Lieferant: Acros) in dem Aceton gelöst. Zu der entstandenen Suspension werden 0,17 ml Bromwasserstoff (w (HBr) = 48%) zugegeben und zweimal unter Rühren evakuiert und mit Argon belüftet. Dann wird 0,5 h unter Argon nachgerührt. Es entsteht eine orange-rote Lösung.

In einem weiteren Kolben werden 10,4 g Trimethylbrenztraubensäuremethylester und 50 ml destilliertes Methanol unter Argon vorgelegt. Diese Lösung wird dreimal unter Rühren evakuiert und mit Argon belüftet. Dann wird die Katalysatorlösung durch eine Hohlnadel aus dem ersten Kolben mit Argon in den zweiten Kolben umgedrückt. Die dabei entstandene gelbe trübe Lösung wird dann in einen 0,3L V₄A-Autoklav, der zuvor 5 mal mit 100 bar Stickstoff (5.0) gespült worden ist, mit Argon umgedrückt.

Unter Rühren (1000U/min) wird der Autoklav auf 50°C erwärmt. Bei 50°C werden 150 bar Wasserstoff aufgedrückt und 24 Stunden unter diesen Bedingungen hydriert. Anschließend wird der Autoklav auf Raumtemperatur abgekühlt, entspannt und mit Stickstoff gespült. Danach wird die Hydrierlösung von den Lösungsmitteln im Vakuum befreit.

Die isolierte Ausbeute beträgt 9,6 g (92 % der Theorie). Gemäß GC ist der Gehalt an (R)-Trimethylmilchsäuremethylester > 99 % mit einem Enantiomerenüberschuss von 98 % ee.

### Beispiel 7

0,52 g Trimethylbrenztraubensäuremethylester und 65ml Methanol werden in einem Kolben unter Argon (5.0) vorgelegt, unter Rühren evakuiert und mit Argon belüftet. Dieser Vorgang wird dreimal wiederholt. Zu der Lösung werden im Argon-Gegenstrom 113,6 mg [Ru (+)-(5,5'-Dichloro-6,6'-dimethoxy-biphenyl-2,2'-diyl)-bis-(diphenylphosphin) Cym J] J zugegeben und dreimal unter Rühren evakuiert und mit Argon belüftet. Die Lösung wird dann in einen 0,3L V₄A-Autoklav, der zuvor fünfmal mit 100 bar Stickstoff (5.0) gespült worden ist, mit Argon umgedrückt.

Unter Rühren (1000U/min) wird der Autoklav auf 50°C erwärmt. Bei 50°C werden 150 bar Wasserstoff aufgedrückt und 24 Stunden unter diesen Bedingungen hydriert. Anschließend wird der Autoklav auf Raumtemperatur abgekühlt, entspannt und mit Stickstoff gespült.

Die Hydrierlösung wird bei 50°C und 200-20 mbar einrotiert. Der Umsatz war laut GC quantitativ zum (R)-Trimethylmilchsäuremethylester mit einem Enantiomerenüberschuss von 95,8 % ee.

### Beispiel 8

0,52 g Trimethylbrenztraubensäuremethylester und 65ml Methanol werden in einem Kolben unter Argon (5.0) vorgelegt, unter Rühren evakuiert und mit Argon belüftet. Dieser Vorgang wird dreimal wiederholt. Zu der Lösung werden im Argon-Gegenstrom 79,9 mg [Ru (+)-(5,5'-Dichloro-6,6'-dimethoxy-biphenyl-2,2'-diyl)-bis-(diphenylphosphin) OAc₂] zugegeben und dreimal unter Rühren evakuiert und mit Argon belüftet. Die Lösung wird dann in einen 0,3L V₄A-Autoklav, der zuvor drei mal mit 100 bar Stickstoff (5.0) gespült worden ist, mit Argon umgedrückt.

Unter Rühren (1000U/min) wird der Autoklav auf 50°C erwärmt. Bei 50°C werden 150 bar Wasserstoff aufgedrückt und 24 Stunden unter diesen Bedingungen hydriert. Anschließend wird der Autoklav auf Raumtemperatur abgekühlt, entspannt und mit Stickstoff gespült. Die Hydrierlösung wird bei 50°C und 200-20 mbar einrotiert.

Der Umsatz ist laut GC quantitativ zum (R)-Trimethylmilchsäuremethylester mit einem Enantiomerenüberschuss von 41,3 % ee.

### Beispiel 9

0,76 g Trimethylbrenztraubensäure (destilliert) und 65ml Methanol werden in einem Kolben unter Argon (5.0) vorgelegt, unter Rühren evakuiert und mit Argon belüftet. Dieser Vorgang wird dreimal wiederholt. Zu der Lösung werden im Argon-Gegenstrom 113,6 mg [Ru (+)-(5,5'-Dichloro-6,6'-dimethoxy-biphenyl-2,2'-diyl)-bis-(diphenylphosphin) Cym J]J zugegeben und dreimal unter Rühren evakuiert und mit Argon belüftet. Die Lösung wird dann in einen 0,3L V₄A-Autoklav, der zuvor fünfmal mit 100 bar Stickstoff (5.0) gespült worden ist, mit Argon umgedrückt.

Unter Rühren (1000U/min) wird der Autoklav auf 50°C erwärmt. Bei 50°C werden 150 bar Wasserstoff aufgedrückt und 24 Stunden unter diesen Bedingungen hydriert. Anschließend wird der Autoklav auf Raumtemperatur abgekühlt, entspannt und mit Stickstoff gespült. Die Hydrierlösung wird bei 50°C und 200-20mbar einrotiert.

Die Ausbeute beträgt nach GC ca. 84 % zur (R)-Trimethylmilchsäure mit einem Enantiomerenüberschuss von 78 % ee.

## Patentansprüche

1. Verfahren zur Herstellung von optisch aktiver Trimethylmilchsäure und/oder ihrer Ester der allgemeinen Formel (I) 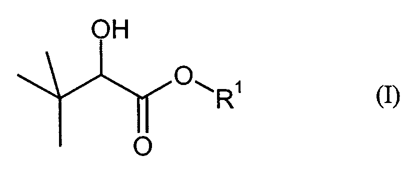 wobei R¹ für Wasserstoff, Alkyl, Aryl, Aralkyl oder Heteroaryl steht,
durch enantioselektive Hydrierung von Trimethylbrenztraubensäure und/oder deren Ester der allgemeinen Formel (II) 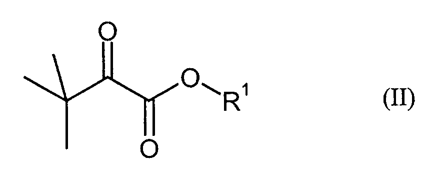 wobei R¹ die für die allgemeine Formel (I) genannten Bedeutungen besitzt,
in Gegenwart eines Katalysators, **dadurch gekennzeichnet, dass** es sich bei dem Katalysator um Edelmetallkomplexe handelt, die optisch aktive Bisphosphine als Liganden enthalten.

2. Verfahren nach Anspruch 1, **dadurch gekennzeichnet, dass** R¹ für H, C₁-C₂₀-Alkyl, bevorzugt C₁-C₁₀-Alkyl, insbesondere Methyl, Ethyl, Propyl, Isopropyl, Butyl, Isobutyl, Sec.-Butyl, Pentyl, Neopentyl oder Isopentyl, C₆-C₁₄-Aryl, bevorzugt C₆-C₁₀-Aryl, insbesondere Phenyl oder Naphthyl, C₇-C₁₅-Aralkyl, bevorzugt C₇-C₁₁-Aralkyl, insbesondere Benzyl, oder C₂-C₁₂-Heteroaryl, bevorzugt C₂-C₉-Heteroalkyl, insbesondere 2-Furyl, 3-Furyl, 2-Pyrrolyl oder 3-Pyrrolyl steht.

3. Verfahren nach Anspruch 2, **dadurch gekennzeichnet, dass** die Alkyl, Aryl, Aralkyl und Heteroalkylreste weiter substituiert sind durch Cl, Br, F, I, C₁-C₄-Alkoxy oder C₁-C₄-Alkyl.

4. Verfahren nach einem oder mehreren der Ansprüche 1-3, **dadurch gekennzeichnet, dass** Katalysatoren mit den folgenden optisch aktiven Bisphosphinen der allgemeinen Formeln (B1)- (B15) eingesetzt werden:
ein Bisphosphin der allgemeinen Formel (B1) 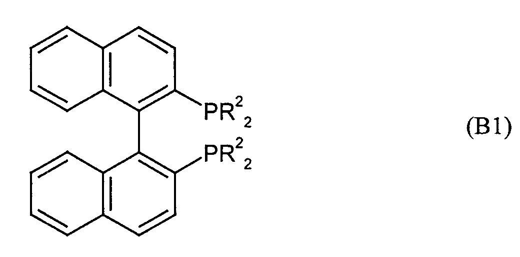 wobei
R² Phenyl, 3-Methylphenyl, 4-Methylphenyl, 3,5-Dimethylphenyl, 4-Methoxyphenyl, 3,5-Dimethyl-4-methoxyphenyl, Cyclohexyl oder Cyclopentyl bedeutet,
oder der allgemeinen Formel (B2) 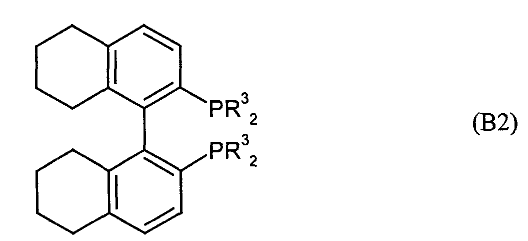 wobei
R³ Phenyl, 4-Methylphenyl, 3,5-Dimethylphenyl, 4-Methoxyphenyl, 3,5-Dimethyl-4-methoxyphenyl oder Cyclohexyl bedeutet,
oder der allgemeinen Formel (B3) 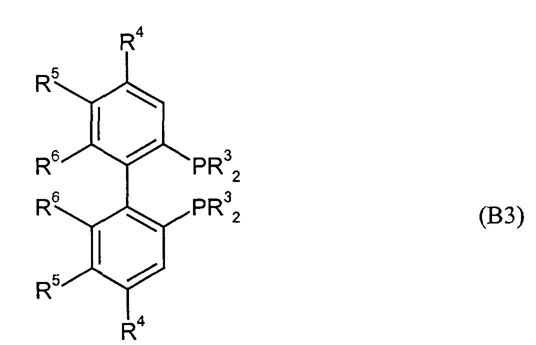 wobei
R³ Phenyl, 4-Methylphenyl, 3,5-Dimethylphenyl, 4-Methoxyphenyl, 3,5-Dimethyl-4-methoxyphenyl oder Cyclohexyl,
R⁴ H, Methyl oder Methoxy,
R⁵ H, Methyl, Methoxy oder Chlor und
R⁶ Methyl, Methoxy oder Trifluoromethyl bedeutet,
oder der allgemeinen Formel (B4) 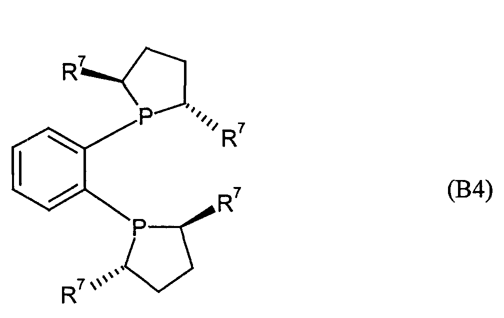 wobei
R⁷ für Methyl, Ethyl, Propyl oder Isopropyl steht,
oder 2,3-Bis-(diphenylphosphino)butan der Formel (B5) 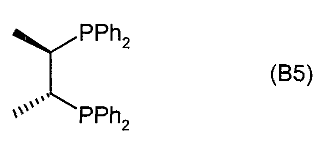 oder 1,2-Bis-(diphenylphosphino)propan der Formel (B6) 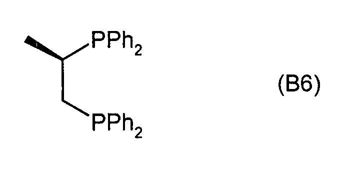 oder 5,6-Bis-(diphenylphosphino)-2-norbornan der Formel (B7) 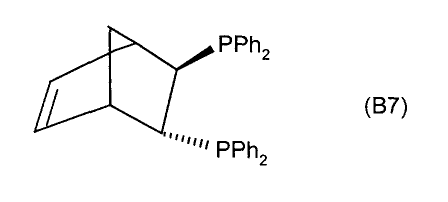 oder 1-substituiertes 3,4-Bis-(diphenylphosphino)pyrrolidin der Formel (B8) 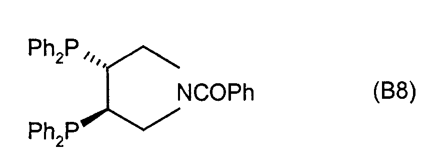 oder 2,4-Bis-(diphenylphosphino)pentan der Formel (B9) 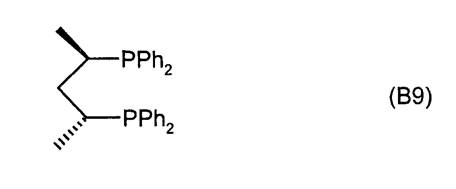 oder 2,3-O-isopropyliden-2,3-dihydroxy-1,4-bis-(diphenylphosphino)butan der Formel (B 10) 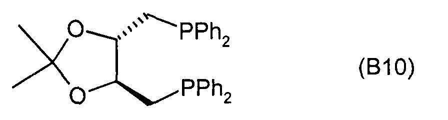 oder 1,2-Bis-[(o-methoxyphenyl)phenylphosphino]ethan der allgemeinen Formel (B11) 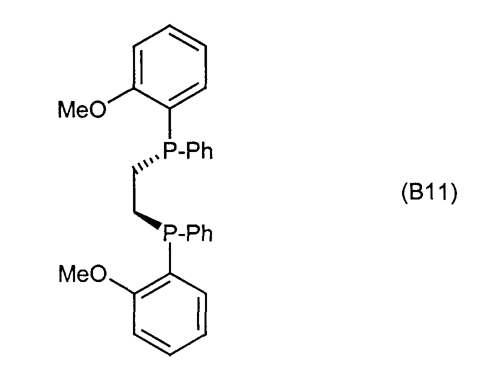 oder 1-[1',2-Bis-(diphenylphosphino)ferrocenyl]ethanol der Formel (B12) 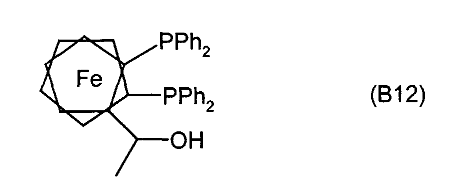 oder 1-tert.Butoxycarbonyl-4-diphenylphosphino-2-diphenylphosphinomethyl-pyrrolidin der Formel (B13) 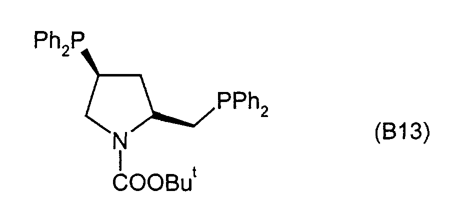 oder der allgemeinen Formel (B14) 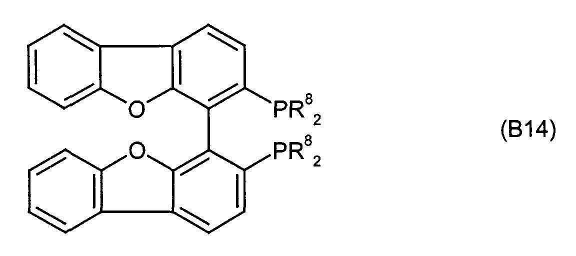 wobei
R⁸ Phenyl, Cyclohexyl, 4-Methylphenyl, 4-Methoxyphenyl, 3,5-Dimethylphenyl, 3,5-Dimethyl-4-methoxyphenyl, 4-tert.-Butyl oder 3,5-di-tert.-Butyl bedeutet,
oder Ferrocenyldiphosphine der allgemeinen Formel (B15) 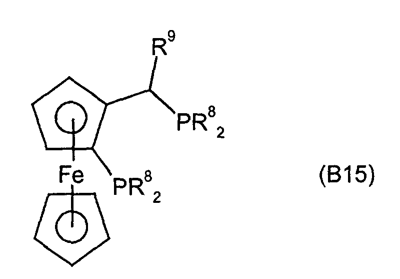 wobei
R⁸ die für Formel B 14 angegebene Bedeutung hat und
R⁹ C₁-C₈-Alkyl, Phenyl oder 1- 3fach mit C₁ - C₄-Alkoxy substituiertes Phenyl bedeutet.

5. Verfahren nach Anspruch 4, **dadurch gekennzeichnet, dass** als Bisphosphin der Formel (B1) 2,2µ-Bis-(diphenylphosphino)-1,1µ-binaphthyl oder 2,2µ-Bis-(di-4-tolylphosphino)-1,1µ-binaphthyl eingesetzt wird.

6. Verfahren nach Anspruch 4, **dadurch gekennzeichnet, dass** die Bisphosphine der Formel (B3) ausgewählt sind aus der Gruppe
(5,5'-Dichloro-6,6'-dimethoxy-biphenyl-2,2'-diyl)-bis-diphenylphosphin (4,4',6,6'-Tetramethyl-5,5'-dimethoxybiphenyl-2,2'-diyl)-bis(diphenylphosphin),
(4,4',6,6'-Tetramethyl-5,5'-dimethoxybiphenyl-2,2'-diyl)-bis(di-p-methoxyphenyl-phosphin),
(4,4',6,6'-Tetratrifluoromethylbiphenyl-2,2'-diyl)-bis(diphenylphosphin)
(4,6-Ditrifluoromethyl-4',6'-dimethyl-5'-methoxybiphenyl-2,2'-diyl)-bis(diphenyl-phosphin),
2-Dicyclohexyl-2'-diphenylphosphino-4,4',6,6'-tetramethyl-5,5 '-dimethoxybiphenyl-2,2'-diyl)-bis(diphenylphosphin),
(6,6'-Dimethyl-2,2'-biphenylen)-bis(diphenylphosphin),
(4,4',6,6'-Tetramethyl-2,2'-biphenylen)-bis(diphenylphosphin),
(3,3 ',6,6 '-Tetramethyl-2,2'-biphenylen)-bis(diphenylphosphin),
(4,4'-Difluoro-6,6'-dimethyl-2,2'-biphenylen)-bis(diphenylphosphin),
(4,4'-bis(Dimethylamino)-6,6'-dimethyl-2,2'-biphenylen)-bis(diphenylphosphin),
(6,6'-Dimethyl-2,2'-biphenylen)-bis(di-p-tolylphosphin),
(6,6'-Dimethyl-2,2'-biphenylen)-bis (di-o-tolylphosphin),
(6,6'-Dimethyl-2,2'-biphenylen)-bis(di-m-fluorophenylphosphin),
1,11-bis(Diphenylphosphino)-5,7-dihydrodibenzo[c,e]oxepin,
(6,6'-Dimethoxybiphenyl-2,2'-diyl)-bis(diphenylphosphin),
(5,5',6,6'-Tetramethoxybiphenyl-2,2'-diyl)-bis(diphenylphosphin),
(6,6'-Dimethoxybiphenyl-2,2'-diyl)-bis(di-p-tolylphosphin) und
(4,4',5,5',6,6'-Hexamethoxybiphenyl-2,2'-diyl)-bis(diphenylphosphin).

7. Verfahren nach Anspruch 4, **dadurch gekennzeichnet, dass** die Bisphosphine der Formel (B4) ausgewählt sind aus der Gruppe
1,2-bis(2,5-Dimethylphosphorano)benzol,
1,2-bis(2,5-Diethylphosphorano)benzol,
1,2-bis(2,5-Dipropylphosphorano)benzol und
1,2-bis(2,5-Diisopropylphosphorano)benzol

8. Verfahren nach einem oder mehreren der Ansprüche 1-7, **dadurch gekennzeichnet, dass** als Edelmetallkomplexkatalysatoren solche auf der Basis von Ruthenium, Rhodium und Iridium eingesetzt werden.

9. Verfahren nach einem oder mehreren der Ansprüche 1-8, **dadurch gekennzeichnet, dass** die durch die allgemeinen Formeln (III)-(X) definierten Rutheniumkomplexe optisch aktiver Bisphosphine eingesetzt werden
Ru₂Cl₄B₂(S) (III)
[Ru Hal Q B]⁺ Y⁻ (IV)
Ru Bₙ OOCR¹⁰OOCR¹¹ (V)
[Ru Hₓ Bₙ]^{m+} Y ^{m-} (VI)
[Ru Hal (PR¹² ₂R¹³)B]⁽²⁺⁾ Hal⁻ ₂ (VII)
[Ru H Hal B₂] (VIII)
[B Ru (acac)₂] (IX)
[B Ru Y₂] (X)
worin
acac Acetylacetonat bedeutet und
B für ein Bisphosphin der allgemeinen Formeln (B1)-(B15) steht,
Hal für Halogen, insbesondere Chlor, Brom oder Iod steht,
R¹⁰ und R¹¹ gleich oder verschieden sind und für C₁-C₉-Alkyl, vorzugsweise C₁-C₄-Alkyl, welches gegebenenfalls substituiert ist durch Halogen, insbesondere Fluor, Chlor oder Brom, stehen oder Phenyl, welches gegebenenfalls durch C₁-C₄-Alkyl substituiert ist, bedeuten oder für eine α-Aminoalkylsäure mit vorzugsweise bis zu 4 C-Atomen stehen, oder R¹⁰ und R¹¹ gemeinsam eine Alkylidengruppe mit bis zu 4 C-Atomen bilden,
R¹² und R¹³ gleich oder verschieden sind und für gegebenenfalls substituiertes Phenyl stehen, vorzugsweise substituiert durch C₁-C₄-Alkyl oder Halogen,
Y für Halogen, ClO₄, BF₄ oder PF₆ steht
Q für einen unsubstituierten oder substituierten Benzolring, bevorzugt p-Cymol, steht,
S für ein tertiäres Amin, bevorzugt Triethylamin, Tri-n-Butylamin oder Pyridin steht,
n und m gleich oder verschieden sind und gleich 1 oder 2 sind
x für 0 oder 1 stehen
wobei in Formel (VI) n für 1 und m für 2 steht, wenn x = 0 ist, und n für 2 und m für 1 steht, wenn x = 1 ist.

10. Verfahren nach einem oder mehreren der Ansprüche 1-9, **dadurch gekennzeichnet, dass** das Verhältnis von Edelmetall zu Bisphosphinen der allgemeinen Formel B1-B15 im Bereich von 0,5 bis 2 Mol, vorzugsweise im Bereich von 0,9 bis 1,1 Mol Ruthenium pro Mol Bisphosphin-Ligand liegt.

11. Verfahren nach einem oder mehreren der Ansprüche 1-10, **dadurch gekennzeichnet, dass** das Verhältnis von Metall in den Komplexen zu den Verbindungen der Formel (II) im Bereich von 1 : 10 bis 1 : 10⁶ und vorzugsweise im Bereich von 1 : 30 bis 1 : 10⁵ liegt.

12. Verfahren nach einem oder mehreren der Ansprüche 1-11, **dadurch gekennzeichnet, dass** die Hydrierung bei einer Temperatur im Bereich von 0-150°C, vorzugsweise im Bereich von 15-100°C, und einem Druck im Bereich von 1 - 250 bar Wasserstoff, vorzugsweise im Bereich von 5 - 200 bar und besonders bevorzugt im Bereich von 10 - 150 bar durchgeführt wird.
